# EUROPÄISCHE PATENTANMELDUNG

(11) **EP 2 138 161 A1**
(43) Veröffentlichungstag der Anmeldung: **30.12.2009**
(21) Anmeldenummer: 09160147.6
(22) Anmeldetag: 13.05.2009
(51) Int. Cl.: A61K 9/00, A61M 5/142

(54) **Implantierbares Wirkstoffreservoir und Vorrichtung mit einem implantierbaren Wirkstoffreservoir**

(30) Priorität: 12.06.2008 DE 102008002396
(71) Anmelder: Biotronik VI Patent AG, 6341 Baar (CH)
(72) Erfinder: Fargahi, Amir, 5242, Birr (CH); Tittelbach, Michael, 90429, Nürnberg (DE); Harder, Claus, 91080, Uttenreuth (DE)
(74) Vertreter: Lindner-Vogt, Karin L.

(57) **Zusammenfassung**

Die Erfindung betrifft ein implantierbares Wirkstoffreservoir (10) mit einem zwischen einem proximalen Ende (14) und einem distalen Ende (16) angeordneten, von einer Hülle (12) umgebenen Hohlraum (28) zur Bevorratung eines oder mehrerer Wirkstoffe (50), mit einer Austrittsöffnung (24) für den oder die Wirkstoffe (50). Über eine Punktionseinrichtung (20) an einem Ende (16) des Werkstoffreservoirs (10) ist der oder sind die Wirkstoffe (50) in ein Freisetzungsgebiet (70) freisetzbar. Die Erfindung betrifft auch eine Vorrichtung (100) mit einem Wirkstoffreservoir (10).

## Beschreibung

Die Erfindung betrifft ein implantierbares Wirkstoffreservoir und eine Vorrichtung mit einem implantierbaren Wirkstoffreservoir nach den Oberbegriffen der unabhängigen Ansprüche.

Es sind implantierbare Vorrichtungen zum regionalen Wirkstoffeinsatz durch transarteriell eingebrachte Wirkstoffdepots bekannt. Diese sind jedoch häufig nicht geeignet, ausreichend große Wirkstoffmengen einzubringen. Herkömmliche Wirkstoffpumpen sind groß, bedürfen einer aktiven Steuerung und müssen daher nach der Anwendung wieder explantiert werden.

Der Erfindung liegt die Aufgabe zugrunde, ein implantierbares Wirkstoffreservoir zu schaffen, das größere Mengen eines Wirkstoffs in ein Freisetzungsgebiet, etwa in ein arterielles Gefäß, abgeben kann.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird gemäß einem ersten Aspekt der Erfindung ein implantierbares Wirkstoffreservoir vorgeschlagen mit einem zwischen einem proximalen Ende und einem distalen Ende angeordneten, von einer Hülle umgebenen Hohlraum zur Bevorratung eines oder mehrerer Wirkstoffe, mit einer Austrittsöffnung für den oder die Wirkstoffe. An einem Ende ist eine Punktionseinrichtung angeordnet, über die der oder die Wirkstoffe in ein Wirkstoff-Freisetzungsgebiet freisetzbar sind. Vorteilhafterweise kann die Freisetzung z.B. in ein Blutgefäß, selbstständig erfolgen. Mittels der Punktionseinrichtung kann eine Gefäßwand durchstoßen werden, um den Wirkstoff innerhalb des Gefäßes freizusetzen.

Bevorzugt kann die Punktionseinrichtung aus einem metallischen Werkstoff gebildet sein. Das Wirkstoffreservoir kann mittels der Punktionseinrichtung z.B. mit einem Gefäßlumen in Kontakt gebracht und mit der Verankerungsvorrichtung fixiert werden. Die Verankerungsvorrichtung ermöglicht das Verkeilen des Wirkstoffreservoirs im Gefäßinnenlumen, etwa durch das Aufklappen von Flügeln, Spreizelementen, Widerhaken und dergleichen. Eine sichere Platzierung des Wirkstoffreservoirs sowie gegebenenfalls ein sicheres Nachfüllen des Wirkstoffreservoirs werden damit ermöglicht. Vorzugsweise kann das distale Ende des Wirkstoffreservoirs einen konischen Verlauf aufweisen, wobei ein bevorzugter Öffnungswinkel des Konus zwischen 2° und 178°, bevorzugt zwischen 10° und 170° liegt.

Das Wirkstoffreservoir kann eine ausreichende Wirkstoffmenge, z.B. im Bereich von 2 mg bis 100 mg enthalten. Es kann sich um einen einzigen Wirkstoff, eine Wirkstoffformulierung oder mehrere Wirkstoffe handeln. Im Folgenden werden diese genannten Varianten verallgemeinernd als Wirkstoff bezeichnet. Wirkstoffe können beispielsweise sein: Immunsuppresiva in der Versorgung transplantierter Organe, Zytostatika in der Versorgung von Tumoren, Insulin und dergleichen. Das Wirkstoffreservoir kann am oder im Freisetzungsgebiet verbleiben und gegebenenfalls nachgefüllt werden. Der Wirkstoff kann vorteilhaft mittels einer kontrollierbaren Elutionskinetik z.B. in ein arterielles Gefäß abgegeben werden, oder aus dem Wirkstoffreservoir diffundieren oder herausgedrückt werden. Elution bezeichnet dabei ein Ab- oder Herauslösen einer oder mehrerer Substanzen aus einer stationären, festen oder flüssigen Phase. Eine mobile Phase, die aus einem oder mehreren Lösungsmitteln besteht, wird an der stationären Phase vorbeigeführt, ausgetragen wird ein Gemisch (Eluat) aus Lösungsmitteln und gelösten Substanzen.

Die Abmessungen des Wirkstoffreservoirs können gering sein, z.B. einen Durchmesser im Bereich von 1 mm und einer Länge im Bereich von wenigen, z.B. 3 mm. Die Abmessungen können an das Einsatzgebiet und den Einsatzzweck leicht angepasst werden. Es können vergleichsweise große Wirkstoffmengen appliziert werden. Die Applikation kann direkt proximal einer Erkrankung in die arterielle Blutzuleitung des erkrankten Gebiets eingebracht werden, so dass der Wirkstoff durch den Blutfluss zum erkrankten Gebiet hintransportiert werden kann. Das Wirkstoffreservoir kann wahlweise mit einem definierten Fördermechanismus ausgestattet sein oder die Wirksubstanz durch Diffusion abgeben. In beiden Fällen kann das Reservoir als nachfüllbares Depot ausgelegt werden. Das Wirkstoffreservoir kann grundsätzlich, zweckmäßigerweise mit Ausnahme der Verankerungsvorrichtung, voll degradierbar ausgelegt werden, so dass eine Explantation nach dem Einsatz vermieden werden kann.

Vorzugsweise ist eine Verankerungsvorrichtung vorgesehen, mit dem das Wirkstoffreservoir an oder in dem Wirkstoff-Freisetzungsgebiet verankerbar ist. Vorzugsweise ist das Wirkstoffreservoir mit seiner Punktionseinrichtung fixierbar. Dies kann direkt erfolgen, wobei eine entsprechende Vorrichtung mit dem Wirkstoffreservoir mitgeführt und im Freisetzungsgebiet ausgelöst wird und/oder es kann ein Gegenelement im Freisetzungsgebiet eingebracht werden und das Wirkstoffreservoir fixieren. Vorteilhaft kann die Verankerung innerhalb eines Gefäßes erfolgen, durch dessen Gefäßwand das Wirkstoffreservoir mit seiner Punktionseinrichtung durchstößt. Das Wirkstoffreservoir kann so gezielt und sicher positioniert über einen längeren Zeitraum am Wirkstoff-Freisetzungsgebiet verbleiben.

Gemäß einer vorteilhaften Weiterbildung können zur Verankerung am oder im Wirkstoff-Freisetzungsgebiet am distalen Ende Widerhaken ausgebildet oder ausbildbar sein. Bevorzugt können dazu im Hohlraum Drähte angeordnet sein, die am distalen Ende zur Verankerung am oder im Wirkstoff-Freisetzungsgebiet ausstoßbar sind. Vorzugsweise können die Drähte wenigstens bereichsweise aus Formgedächtnismaterial, etwa einer Nickel-Titan-Legierung, insbesondere Nitinol, gebildet sein. Vorteilhafterweise können die Drähte beim Einführen des Wirkstoffreservoirs innerhalb desselben so angeordnet sein, dass diese am distalen Ende nicht und am proximalen Ende geringfügig überstehen. Zweckmäßigerweise kann das proximale Ende mit einer Abdeckung versehen sein, welche von den Drähten durchsetzt ist. Ist das Wirkstoffreservoir am gewünschten Freisetzungsgebiet angelangt, können die Drähte vom proximalen Ende her zum distalen Ende hin geschoben werden, so dass diese aus der Austrittsöffnung ragen. Aufgrund der Eigenschaften des Formgedächtnismaterials klappen die Drähte dann an ihrem freien Ende um und verankern das Wirkstoffreservoir sicher im Freisetzungsgebiet.

Günstigerweise kann die Hülle des Wirkstoffreservoirs aus einem degradierbaren Polymer gebildet sein, so dass ein Explantieren des Wirkstoffreservoirs zu einem späteren Zeitpunkt entfallen kann.

Eine vorteilhafte Weiterbildung sieht vor, dass am proximalen Ende der Hülle ein quellfähiges Material angeordnet sein kann, das durch Aufquellen einen zwischen dem Material und dem distalen Ende befindlichen Wirkstoff aus der Austrittsöffnung drängt. Bevorzugt kann ein poröser Verschluss die Hülle am proximalen Ende verschließen. Bevorzugt kann das quellfähige Material durch ein degradierbares Polymer gebildet sein. Durch den porösen Verschluss kann Wasser am proximalen Ende in das Wirkstoffreservoir eindringen und ein Aufquellen des quellfähigen Materials bewirken. Dies hat den Vorteil, dass das Aufquellen des quellfähigen Materials durch die Porosität, etwa Anzahl und/oder Größe der Poren, gezielt gesteuert werden kann. Dadurch kann wiederum das Austreten des Wirkstoffs aus dem Wirkstoffreservoir in seiner Geschwindigkeit beeinflusst werden. Mit Vorteil kann zwischen dem quellfähigen Material und dem Wirkstoff eine verschiebliche Trennwand angeordnet sein, welche eine Mischung des quellfähigen Materials und des Wirkstoffs einerseits verhindert und andererseits ein Austreten des Wirkstoffs definiert steuert, da ein möglicherweise inhomogenes Aufquellen durch die Trennwand in seiner Wirkung auf den Wirkstoff homogenisiert wird.

Günstigerweise kann am proximalen Ende ein Ansatz ausgebildet sein, der sich von der Hülle weg im Durchmesser aufweitet. Bevorzugt kann der Ansatz wenigstens bereichsweise für Röntgenstrahlen undurchlässig ausgebildet sein. Der Ansatz erleichtert vorteilhafterweise ein Ankoppeln eines Katheters oder einer Kanüle, mit dem das Wirkstoffreservoir befüllt oder nachgefüllt werden kann. Der sich proximal trichterförmig erweiternde Ansatz erleichtert das Einführen einer Spitze des Katheters in das Wirkstoffreservoir. Beim Heranführen des Katheders an das Wirkstoffreservoir führt der Ansatz die Spitze des Katheders zum proximalen Ende des Wirkstoffreservoirs, das vorteilhafterweise mit einer Membran, z.B. einer Teflonmembran, verschlossen sein kann. Die Spitze des Katheders kann an seinem distalen Ende mit einer Spritzennadel ausgestattet sein, welche dann die Membran durchstoßen und das Wirkstoffreservoir befüllen kann. Beim Zurückziehen der Spritzennadel nach dem Befüllen kann die Membran die Durchstoßungsstelle verschießen. Ist der Ansatz ganz oder wenigstens teilweise röntgendicht ausgebildet, kann das Auffinden des Wirkstoffreservoirs und des Ansatzes stark vereinfacht werden und z.B. in Echtzeit verfolgt werden.

Gemäß einem weiteren Aspekt der Erfindung wird eine Vorrichtung mit einem Wirkstoffreservoir mit wenigstens einem der vorstehend beschriebenen Merkmale vorgeschlagen, bei der wenigstens ein Koppelelement und/oder Stabilisierungselement zum Koppeln des Wirkstoffreservoirs am oder im Wirkstoff-Freisetzungsgebiet vorgesehen ist. Dadurch kann eine zuverlässige Fixierung des Wirkstoffreservoirs erreicht werden. So kann ein Koppelelement z.B. in ein Blutgefäß eingebracht werden, das mit dem Wirkstoffreservoir, insbesondere mit seiner Punktionseinrichtung, verrastbar ist. So kann ein spitz zulaufendes distales Ende des Wirkstoffreservoirs beispielsweise über einen einfachen Schnappmechanismus des Koppelelements an diesem festgelegt werden. Ein solches Koppelelement kann vorteilhaft sein, wenn etwa eine Gefäßwand durchstoßen werden muss, die besonders geschont werden soll. Gleichzeitig bietet das Wirkstoffreservoir mehr Volumen für einen Wirkstoff, da auf eine Verankerungsvorrichtung verzichtet werden kann, die im oder mit dem Wirkstoffreservoir eingeführt werden muss.

Vorteilhaft können quer zu einer Längserstreckung des Wirkstoffreservoirs schalenförmige oder rechenartige Flügelelemente als Stabilisierungs- oder Koppelelemente vorgesehen sein. Die Flügelelemente können mit einem Scharnier miteinander Verbunden sein und mit ihren freien Enden aufeinander zu bewegt werden, um ein Gewebestück, etwa einen Abschnitt eines Blutgefäßes, zwischen sich einzuschließen. Das Wirkstoffreservoir stößt mit seinem distalen Ende durch die Flügelelemente, z.B. im Bereich einer Trennstelle oder eines Scharniers. Die Flügelelemente können das Gewebestück, etwa einen Blutgefäßabschnitt, vorteilhaft stabilisieren. Die Flügelelemente können als aufgeschnittene Rohre oder in der Art eines Rechens ausgebildet sein. Sind die Flügelelemente geschlossen und das umschlossenen Freisetzungsgebiet somit stabilisiert, kann das Wirkstoffreservoir mit seinem Punktionsbesteck herangeführt und mit etwaigen Koppelelementen im Freisetzungsgebiet verbunden werden, oder es kann eine reservoirseitige Verankerungsvorrichtung ausgelöst werden. Vorteilhaft kann zur Verankerung wenigstens ein Koppelelement im Freisetzungsgebiet vorgesehen sein, mit dem das distale Ende des Wirkstoffreservoirs koppelbar ist.

Die Erfindung ist nachfolgend beispielhaft anhand von in Zeichnungen dargestellten Ausführungsbeispielen näher erläutert. Es zeigen in schematischer Darstellung:
- Fig. 1a-1d: Schnittdarstellungen eines bevorzugten Wirkstoffreservoirs gemäß einer ersten Ausgestaltung der Erfindung mit einer Verankerungsvorrichtung aus einem Formgedächtnismaterial mit einem Detail (Fig. 1b) und in unterschiedlichen Stadien des Einsetzens des Wirkstoffreservoirs (Fig. 1a, 1c, 1d);
- Fig. 2a-2c: bevorzugte Vorrichtungen mit einem Wirkstoffreservoir und Koppelelementen mit halbschalenartigen (Fig. 2a, 2b) und rechenartigen (Fig. 2c) Flügelelementen; und
- Fig. 3: einen Schnitt durch ein bevorzugtes Wirkstoffreservoir mit einem quellfähigen Material zum Austreiben eines Wirkstoffs aus dem Wirkstoffreservoir; und
- Fig. 4: einen Schnitt durch ein bevorzugtes Wirkstoffreservoir mit einer Einführhilfe zum Befüllen des Wirkstoffreservoirs.

In den Figuren sind funktionell gleiche oder gleich wirkende Elemente jeweils mit den selben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

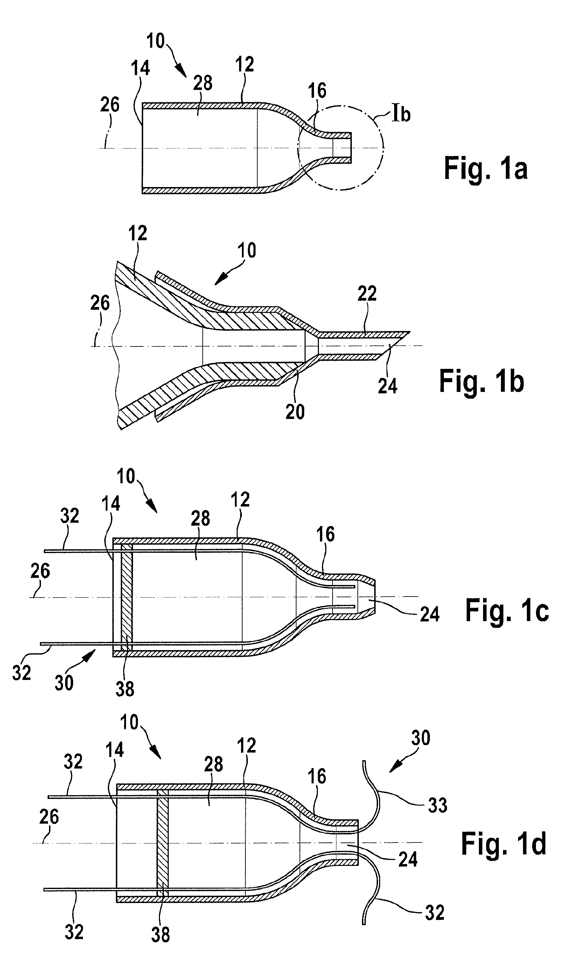

Zur Erläuterung der Erfindung zeigen die Fig. 1a-1d ein erstes Ausführungsbeispiel eines bevorzugten implantierbaren Wirkstoffreservoirs 10 mit einem zwischen einem proximalen Ende 14 und einem distalen Ende 16 angeordneten, von einer Hülle 12 umgebenen Hohlraum 28 zur Bevorratung eines oder mehrerer Wirkstoffe 50. Das Wirkstoffreservoir 10 weist eine Austrittsöffnung 24 für den oder die Wirkstoffe 50 auf. Es ist eine Verankerungsvorrichtung 30 vorgesehen, mit dem die Hülle 12 bzw. das Wirkstoffreservoir 10 an oder in einem Wirkstoff-Freisetzungsgebiet (nicht dargestellt) verankerbar ist. Das Wirkstoffreservoir 10 kann insbesondere durch extravaskuläres Gewebe an ein arterielles Gefäß herangebracht werden, welches das Freisetzungsgebiet bildet. Wirkstoff aus dem Wirkstoffreservoir 10 kann in den Blutstrom freigesetzt, durch diesen abtransportiert und zu seinem Wirkungsort hintransportiert werden.

Das Wirkstoffreservoir 10 ist entlang einer Längserstreckung 26 zylinderförmig ausgebildet und läuft an seinem distalen Ende 16 spitz zu. Am distalen Ende 16 weist das Wirkstoffreservoir 10 die Austrittsöffnung 24 auf. Die Hülle 12 kann vorzugsweise aus einem degradierbaren Polymer ausgebildet sein. An dem distalen Ende 16 ist eine Punktionseinrichtung 20 mit einer Spritzennadel 22 vorgesehen, welche die Austrittsöffnung 24 aufweist. Die Punktionseinrichtung 20 kann in die Hülle integriert sein, oder sie kann, wie in Fig. 1b als Detailansicht der Fig. 1a dargestellt ist, auf die Hülle 12 aufgesetzt sein. Im Hohlraum 28 befindet sind der Wirkstoff 50, der durch die Spritzennadel 22 aus dem Wirkstoffreservoir 10 austreten kann. Mit der Punktionseinrichtung 20 dringt das Wirkstoffreservoir 10 z.B. durch eine Gefäßwand (nicht dargestellt) und ragt dort in das Gefäßinnenlumen hinein, in das der Wirkstoff 50 abgegeben und in einem dort vorhandenen Blutstrom weitertransportiert werden kann.

Um eine Verankerung des Wirkstoffreservoirs 10 im Wirkstoff-Freisetzungsgebiet zu ermöglichen, sind im Hohlraum 28 Drähte 32 angeordnet, die vorzugsweise aus einem Formgedächtnismaterial, z.B. einer Nickel-Titanlegierung (Nitinol) ausgeführt werden. Die Drähte 32 können innenumfänglich an der Hülle 12 angeordnet sein und am proximalen Ende 14 eine ansonsten vorzugsweise dichte Scheibe 38 als Abdeckung durchstoßen. Vor dem Verankern des Wirkstoffreservoirs 10 können die Drähte 32 über das proximale Ende 14 in proximaler Richtung überstehen, z.B. um 1-5 mm, vorzugsweise 1.5 mm (Fig. 1c). Die Scheibe 38 dichtet das proximale Ende 14 gegen die Umgebung ab und ist vorzugsweise entlang der Längserstreckung 26 des Wirkstoffreservoirs 10 verschieblich angeordnet. Die Scheibe 38 kann vorteilhafterweise aus einem degradierbaren Polymer gebildet sein. Zweckmäßigerweise kann auch die Hülle 12 aus einem degradierbaren Polymer gebildet sein und die Punktionseinrichtung 20 aus einem metallischen Werkstoff.

Zur Verankerung am oder im Wirkstoff-Freisetzungsgebiet werden die Drähte 32, nachdem das Wirkstoffreservoir 10 am gewünschten Ort positioniert ist und z.B. die Punktionseinrichtung 20 des Wirkstoffreservoirs 10 die Gefäßwand durchstoßen hat, aus dem distalen Ende 16 bzw. der Öffnung 24 geschoben, indem die Scheibe 38 zum distalen Ende 16 hin bewegt wird, wobei sie aufgrund der Eigenschaften des Formgedächtnismaterials nach außen umklappen und quer zur Längsachse 26 am distalen Ende 16 abstehende Widerhaken 33 ausbilden, mit denen das Wirkstoffreservoir 10 im Gefäß verkeilt bzw. verankert wird.

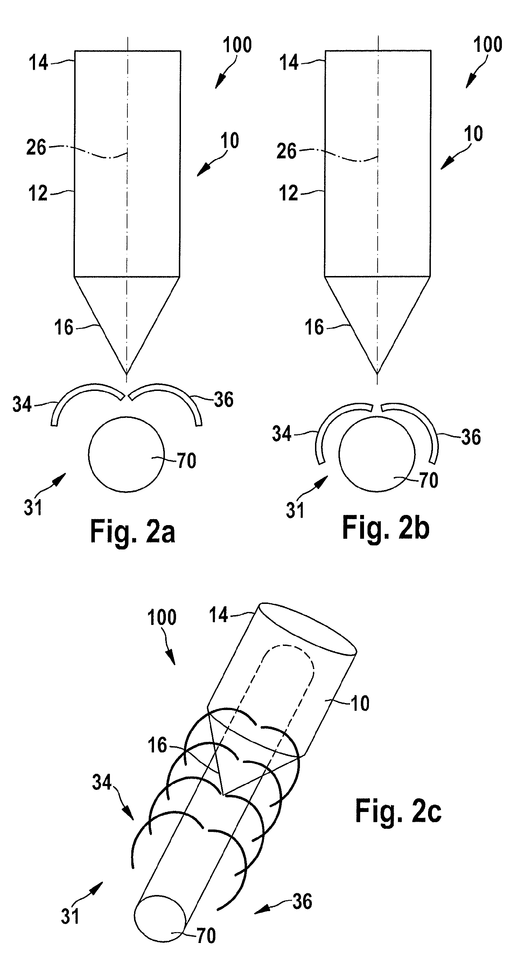

Günstige Varianten bevorzugter Vorrichtungen 100 mit einem Wirkstoffreservoir 10 sind in den Fig. 2a-2c dargestellt. Ein bevorzugtes Ausführungsbeispiel des Wirkstoffreservoirs 10 wurde bereits in den Fig. 1a-1d im Detail beschreiben, so dass zur Vermeidung unnötiger Wiederholungen auf diese Beschreibung verwiesen wird.

Zum Stabilisieren der Positionierung des Wirkstoffreservoirs 10 ist gemäß einer bevorzugten Variante ein Stabilisierungselement 31 vorgesehen, z.B. in der Art von Halbschalen 34, 36 eines längs seiner Längserstreckung aufgeschnittenen Röhrchens, das quer zur Längsachse 26 des Wirkstoffreservoirs 10 ausgerichtet ist. Die beiden Halbschalen 34, 36 werden vor der Applikation des Wirkstoffreservoirs 10 auf beiden Seiten des Zielgefäßabschnitts (Freisetzungsgebiet 70) positioniert (Fig. 2a). Über einen von außen auslösbaren Mechanismus schließen sich die Halbschalen 34, 36 um den Zielgefäßabschnitt (Fig. 2b), indem sie z.B. um ein die Halbschalen in axialer Richtung verbindendes Scharnier geschwenkt werden. Der derart stabilisierte Gefäßabschnitt kann dann vom Wirkstoffreservoir 10 punktiert werden und z.B. eine Verankerungsvorrichtung 30, wie in Fig. 1a-1d beschrieben, ausgelöst werden. Es wird eine stabile Fixierung des Wirkstoffreservoirs 10 im Freisetzungsgebiet 70 ermöglicht.

Die Halbschalen 34, 36 können aus Vollmaterial gebildet sein, oder sie können rechenartig mit einzelnen kreisbogenartig geformten, in axialer Richtung beabstandeten Segmenten ausgebildet sein, wie in Fig. 2c angedeutet ist, oder sie können z.B. aus einem Gewebe oder Gestrick gebildet sein.

Es kann auch zusätzlich oder alternativ ein Koppelelement innerhalb des Zielgefäßabschnitts (Freisetzungsgebiet 70) vor der Applikation des Wirkstoffreservoirs 10 positioniert werden, mit dem das Wirkstoffreservoir 10 durch einen einfachen Schnappmechanismus verrasten kann. In diesem Fall kann gegebenenfalls auf eine Verankerungsvorrichtung 30 (Fig. 1c-1d) verzichtet werden.

Der Wirkstoff 50 kann aus dem Wirkstoffreservoir 10 auf unterschiedliche Arten freigesetzt werden. Das Wirkstoffreservoir 10 setzt den Wirkstoff nur durch Diffusion aus dem Hohlraum 28 durch die Spritzennadel 22 frei; das proximale Ende 14 des Wirkstoffreservoirs 10 ist geschlossen. Das Wirkstoffreservoir 10 kann an seinem proximalen Ende 14 eine Membran 42, vorzugsweise eine Teflonmembran, aufweisen, über die bei Bedarf mit einem Befüllungskatheter durch Durchstechen der Membran Wirkstoff nachgefüllt werden kann. Das Wirkstoffreservoir 10 kann mit einem selbstständigen Fördermechanismus ausgestattet sein, so dass ohne externe Betätigung Wirkstoff 50 in das Freisetzungsgebiet 70 abgeben kann..

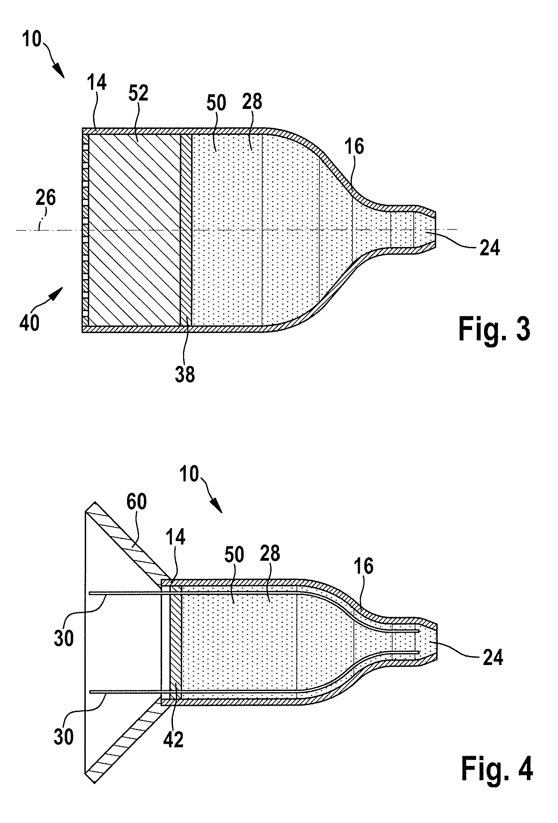

Ein bevorzugter selbstständiger Fördermechanismus ist in Fig. 3 skizziert. Am proximalen Ende 14 der Hülle 12 des Wirkstoffreservoirs (Fig. 1a-1d) ist im Hohlraum 28 des Wirkstoffreservoirs 10 ein quellfähiges Material 52 angeordnet, das durch Einwirken von Wasser aufquillt und so ein größeres Volumen einnimmt als im nicht aufgequollenen Zustand. Durch das Aufquellen wird ein zwischen dem Material und dem distalen Ende 16 befindlichen Wirkstoff aus der Austrittsöffnung 24 gedrängt. Zwischen dem quellfähigen Material 52 und dem Wirkstoff 50 ist eine Scheibe 38 angeordnet, die z.B. von Drähten 32 einer Verankerungsvorrichtung 30 (nicht dargestellt, s. Fig.1c-1d), durchstoßen ist. Wirkt vom proximalen Ende 14 her ein Druck auf die Scheibe 38, drückt diese eine dem Druck entsprechende Menge des Wirkstoffs 50 aus der Öffnung 24.

Das quellfähige Material 52 kann z.B. aus Zellulose gebildet sein. Ein poröser Verschluss 40 am proximalen Ende 14, der z.B. als gelochte Scheibe ausgebildet sein kann, verschließt die Hülle 12 bzw. das Wirkstoffreservoir 10. Durch die Porosität des Verschlusses 40 kann gezielt ein gewünschter Flüssigkeitseintrag in das quellfähige Material 52 eingestellt und damit die Materialquellung reguliert werden. Über die Materialquellung des quellfähigen Materials 52 kann wiederum die Menge des aus der Öffnung 24 austretenden Wirkstoffs 50 eingestellt werden. Dadurch, dass das Material mit vorbekannter Kinetik aufquellen kann, lässt sich der Wirkstoffaustritt genau steuern.

Die Fig. 4 zeigt eine bevorzugte Ausgestaltung eines Wirkstoffreservoirs 10, bei dem ein nachträgliches Befüllen des Wirkstoffreservoirs 10 und/oder ein Nachfüllen des Wirkstoffs 50 möglich ist.

Am proximalen Ende 14 ist ein Ansatz 60 ausgebildet, der sich vom proximalen Ende 14 weg im Durchmesser trichterförmig aufweitet. Der Ansatz 60 ist wenigstens bereichsweise für Röntgenstrahlen undurchlässig oder ganz aus einem für Röntgenstrahlen undurchlässigen Material gebildet und kann auf diese Weise leicht von außen geortet werden. Das proximale Ende 14 ist mit einer Membran 42, vorzugsweise einer Teflonmembran, verschlossen. An die Membran 42 schließt sich in proximaler Richtung der Ansatz 60 an. Der sich trichterförmig erweiternde Ansatz 60 dient dem Positionieren und Führen eines Befüllungskatheters (nicht dargestellt) mit einer distalen Spritzennadel, mit dem das Wirkstoffreservoir 10 im eingesetzten Zustand mit Wirkstoff 50 befüllt werden kann, indem die Spritzennadel die Membran 42 durchdringt. Beim Rückzug der Spritzennadel verschließt sich die Membran 42 wieder, so dass der Hohlraum 28 des Wirkstoffreservoirs 10 nach außen wieder abgedichtet ist.

## Patentansprüche

1. Implantierbares Wirkstoffreservoir (10) mit einem zwischen einem proximalen Ende (14) und einem distalen Ende (16) angeordneten, von einer Hülle (12) umgebenen Hohlraum (28) zur Bevorratung eines oder mehrerer Wirkstoffe (50), mit einer Austrittsöffnung (24) für den oder die Wirkstoffe, **dadurch gekennzeichnet, dass** an einem Ende (16) eine Punktionseinrichtung (20) angeordnet ist, über die der oder die Wirkstoffe (50) in ein Freisetzungsgebiet (70) freisetzbar ist.

2. Wirkstoffreservoir nach Anspruch 1, **dadurch gekennzeichnet, dass** eine Verankerungsvorrichtung (30) vorgesehen ist, mit dem die Hülle (12) an oder in einem Wirkstoff-Freisetzungsgebiet (70) verankerbar ist.

3. Wirkstoffreservoir nach Anspruch 1 oder 2, **dadurch gekennzeichnet, dass** zur Verankerung am oder im Wirkstoff-Freisetzungsgebiet (70) am distalen Ende (16) Widerhaken (33) ausgebildet oder ausbildbar sind.

4. Wirkstoffreservoir nach Anspruch 3, **dadurch gekennzeichnet, dass** im Hohlraum (28) Drähte (32) angeordnet sind, die am distalen Ende (16) zur Verankerung am oder im Wirkstoff-Freisetzungsgebiet (70) ausstoßbar sind.

5. Wirkstoffreservoir nach Anspruch 3 oder 4, **dadurch gekennzeichnet, dass** die Drähte (32) wenigstens bereichsweise aus Formgedächtnismaterial gebildet sind.

6. Wirkstoffreservoir nach einem der vorhergehenden Ansprüche, **dadurch gekennzeichnet, dass** die Punktionseinrichtung (20) aus einem metallischen Werkstoff gebildet ist.

7. Wirkstoffreservoir nach einem der vorhergehenden Ansprüche, **dadurch gekennzeichnet, dass** die Hülle (12) aus einem degradierbaren Polymer gebildet ist.

8. Wirkstoffreservoir nach einem der vorhergehenden Ansprüche, **dadurch gekennzeichnet, dass** im Hohlraum (28) am proximalen Ende (14) ein quellfähiges Material angeordnet ist, das durch Aufquellen einen zwischen dem quellfähigen Material und dem distalen Ende (16) befindlichen Wirkstoff (50) aus der Austrittsöffnung (24) drängt.

9. Wirkstoffreservoir nach Anspruch 8, **dadurch gekennzeichnet, dass** ein poröser Verschluss (40) am proximalen Ende (14) den Holraum (28) verschließt.

10. Wirkstoffreservoir nach einem der vorhergehenden Ansprüche, **dadurch gekennzeichnet, dass** am proximalen Ende (14) ein Ansatz (60) ausgebildet ist, der sich von der Hülle (12) weg im Durchmesser aufweitet.

11. Wirkstoffreservoir nach Anspruch 10, **dadurch gekennzeichnet, dass** der Ansatz (60) wenigstens bereichsweise für Röntgenstrahlen undurchlässig ausgebildet ist.

12. Vorrichtung mit einem Wirkstoffreservoir (10) nach einem der vorhergehenden Ansprüche, **dadurch gekennzeichnet, dass** wenigstens ein Koppelelement und/oder Stabilisierungselement (31) zum Koppeln des Wirkstoffreservoirs (10) am oder im Wirkstoff-Freisetzungsgebiet (70) vorgesehen ist.

13. Vorrichtung nach Anspruch 12, **dadurch gekennzeichnet, dass** zur Stabilisierung quer zu einer Längserstreckung (26) des Wirkstoffreservoirs (10) Flügelelemente (34, 36) vorgesehen sind, die aufeinander zu bewegbar sind, um ein Gewebestück zwischen sich zu umschließen, an die das Wirkstoffreservoir (10) mit seinem distalen Ende (16) ankoppelbar ist.

14. Vorrichtung nach Anspruch 12 oder 13, **dadurch gekennzeichnet, dass** zur Verankerung ein Koppelelement im Freisetzungsgebiet (70) vorgesehen ist, mit dem das distale Ende (16) verrastbar ist.
